Europäisches Patentamt

European Patent Office

Office européen des brevets

(11) Publication number: **0 427 429 A2**

# EUROPEAN PATENT APPLICATION

(21) Application number: **90311707.5**

(22) Date of filing: **25.10.90**

(51) Int. Cl.⁵: **A61M 25/10**

(30) Priority: **25.10.89 US 426879**

(43) Date of publication of application:
**15.05.91 Bulletin 91/20**

(84) Designated Contracting States:
**BE DE ES FR GB IT NL**

(71) Applicant: **C.R. BARD, INC.**
**730 Central Avenue**
**Murray Hill New Jersey 07974(US)**

(72) Inventor: **Daignault, Kenneth J., Jr.**
**15 Quinapoxit Lane**
**Jefferson, Massachusetts 01522(US)**
Inventor: **McNamara, Edward I.**
**89 High Street**
**Chelmsford, Massachusetts 01820(US)**
Inventor: **Saunders, Myles L.**
**8635 West Third Street**
**Los Angeles, California 90048(US)**

(74) Representative: **Woodward, John Calvin et al**
**VENNER SHIPLEY & CO. 368 City Road**
**London EC1V 2QA(GB)**

(54) Occluding catheter for treating cerebral arteries.

(57) An occlusion catheter for use during therapeutic intervention of a blood vessel for flushing away debris including an elongated guide catheter (20) sized to be introduced into the blood vessel, an occlusion balloon (12) located at or near the distal end of the catheter and an inflation lumen (18) integral with the guide catheter and communicating with the interior of the balloon enabling the balloon to be inflated to form a seal between the balloon and the blood vessel wall. The balloon is capable of being expanded to at least 5 times its deflated diameter when inflated.

## OCCLUDING CATHETER FOR TREATING CEREBRAL ARTERIES

The present invention relates to devices for treating stenoses in cerebral arteries.

A stenosed blood vessel can be life-threatening and often requires therapeutic intervention. Non-surgical intervention procedures have been used as a treatment for stenosed arteries with increasing regularity in recent years. Examples of non-surgical intervention include treatments such as balloon angioplasty, mechanical removal, laser fiber-optical treatment and localized chemical treatment.

The foregoing procedures typically involve the percutaneous introduction of the treatment device into the lumen of the artery, via a catheter. For example, balloon angioplasty involves the use of a special catheter having a dilatation balloon at its distal end. The catheter is inserted percutaneously into the patient's arterial system and is advanced and manipulated to place the dilatation balloon within the stenosis in the artery. The dilatation balloon then is inflated under substantial pressure to press the plaque and plaque-laden arterial wall radially outwardly to enlarge the stenosed region of artery. When successful, this dilatation procedure may avoid major surgery.

Treatment of cerebral arteries poses unusual risks. The above-listed forms of non-surgical treatments potentially involve the release of debris into the patient's circulatory system as the stenosis is manipulated during the treatment. Such debris may be carried distally by the circulating blood to more narrowed vessels of the brain. If the debris occludes such narrowed vessels it may cause serious trauma, including stroke. Fear of such strokes thus far has prevented the widespread use of balloon angioplasty in the cerebral vascular system. The previous attempts to perform cerebral percutaneous transluminal angioplasty have been limited to a small number of dilatations of proximal, extra-cranial vessels, and rare cases of distal intra-cranial vessels.

### Summary of the Invention

The present invention permits treatment of both external carotid arteries and internal carotid arteries while eliminating/reducing the risk of embolic stroke by preventing debris from traveling distally toward the Circle of Willis and other critical arteries. An occlusion device of special design is placed in the cerebral artery proximal to the treatment site. The device is used to briefly occlude the circulating blood flow in the artery at the time the obstructive lesion is manipulated. Passages within the cerebral circulation system cause blood to flow retrograde towards the occlusion device, and debris may be flushed safely away from the treatment site either through an external carotid artery or through a central shaft of the occlusion device.

The cerebral circulation system generally may be seen as divided into two sets of contralateral arteries, one side feeding the left side of the brain and the other feeding the right side. A large number of major communicating vessels connects these contralateral arteries. As such, if pressure becomes low enough on a given side, the physiological pressure on the contralateral side is sufficient to cause blood to flow across the communicating vessels and in a retrograde fashion towards the low pressure source. According to the invention, by temporarily occluding the natural antegrade flow in a cerebral vessel, this retrograde effect can be artificially induced and used to flush post-treatment debris safely away from the treatment site.

The cerebral circulatory system includes a common cerebral artery which bifurcates into an internal carotid artery and an external carotid artery. The external carotid artery channels blood distally to regions such as the face. The internal carotid artery channels blood distally to the more narrowed blood vessels supplying the brain, including the vessels forming the Circle of Willis and other critical arteries. These vessels supply blood to regions of the brain critical to normal function. The temporary disruption of the flow of blood to the brain via these more narrowed vessels may have a devastating effect.

When a stenosis occurs in either or both of the internal and external carotid arteries at or very close to the bifurcation, then there is no room to occlude proximally of the stenosis the artery in which the stenosis occurs. Instead, the common carotid artery must be occluded proximal of the bifurcation. When the common carotid artery is occluded proximal to the bifurcation, the resulting blood flow pattern may be of one of two forms. The blood may flow retrograde in the external carotid artery toward and distally through the internal carotid artery, or in the opposite direction, retrograde in the internal carotid artery toward and distally through the external carotid artery. The direction of blood flow differs from individual to individual and cannot be predicted. The present invention takes into account this phenomenon and provides methods and devices for establishing retrograde flow in the internal carotid artery to prevent debris from flowing distally toward the Circle of Willis or other critical arteries, even if flow is antegrade in the internal carotid artery when the common carotid artery first is occluded.

The catheter system for achieving retrograde flow and treatment employs at least two components, an intervention component, such as a conventional dilatation balloon and an occlusion component. The occlusion component may be used to circumferentially seal a vessel lumen and promote retrograde flow of blood away from the Circle of Willis or other critical arteries and toward either an external carotid artery or a central shaft of the occlusion component. The circumferential seal also prevents leakage of the debris at the occlusion component-vessel wall interface. The intervention component and the occlusion component may be mounted together on a single catheter or separately on two-coaxial catheters. Two occlusion components may be required to establish the proper retrograde flow.

Preferably, the occlusion component includes an elastic balloon carried on a guide catheter, the occlusion balloon being secured at or near the distal end of the guide catheter. Preferably the balloon is capable of expanding at least about 500%, and the guide catheter preferably has a through lumen with an inner diameter of about .068". An inflation lumen passes axially through the guide catheter and communicates with the interior of the occlusion balloon. An efflux port in the guide catheter is located just distally of the occlusion balloon and communicates with a central lumen that passes axially through the guide catheter, enabling fluid and debris to be carried through the guide catheter towards its proximal end.

The intervention component may be a single component such as a dilatation balloon catheter or may be multiple components such as, for example, two dilatation balloon catheters. The dilatation balloon catheter(s) may be delivered to the stenosis through a lumen extending axially through the guide catheter. According to this construction, the dilatation balloon(s) may be placed independently of the occlusion balloon.

The catheter of the invention can be used in a method for treating a stenosis disposed in both an internal carotid artery and an external carotid artery at a location close to the bifurcation. Preferably, a guide catheter carrying an occlusion balloon is advanced into the common carotid artery until the occlusion balloon is positioned at a location just proximal to the bifurcation of the external carotid artery and the internal carotid artery. The guide catheter has a valve disposed at its proximal end to prevent blood from flowing down its central lumen. The occlusion balloon then is inflated to occlude the flow of blood through the common carotid artery. It is then determined whether blood is flowing retrograde in the external carotid artery toward the internal carotid artery or retrograde in the internal carotid artery toward the external carotid artery. The valve in the guide catheter then is opened, and an intervention component, preferably a dilatation balloon catheter, is introduced through the central lumen of the guide catheter and is positioned across the stenosis in the internal carotid artery. Another intervention component, preferably a dilatation balloon catheter, is introduced in the same manner and is positioned across the stenosis in the external carotid artery. The valve then is closed and both dilatation balloons are inflated according to standard treatment procedures. Assuming that occlusion of the common carotid artery resulted in blood flow retrograde from the internal carotid artery to the external carotid artery, the dilatation balloon in the internal and external carotid arteries are deflated. This reestablishes retrograde flow in the internal carotid artery toward the external carotid artery. Consequently, all debris will flow in a direction away from the Circle of Willis and other critical arteries and distally out the external carotid artery. After flushing, the occlusion balloon may be deflated and removed, thus reestablishing normal antegrade flow.

The catheter of the present invention can also be used in a second method for treating a stenosis located in both an internal carotid artery and an external carotid artery at a location near the bifurcation. This method is employed when occlusion of the common carotid artery results in blood flowing retrograde in the external carotid artery toward and distally through the internal carotid artery. The common cerebral artery is occluded, preferably with a guide catheter carrying an occlusion balloon. After determining the direction of blood flow, two intervention components, preferably balloon dilatation catheters, are positioned, one each in the internal and external carotid arteries. The balloons then are inflated to treat the stenosis. The dilatation balloon in the internal carotid artery then is deflated while the dilatation balloon in the external carotid artery remains inflated to establish retrograde pressure in the internal carotid artery. A valve at the proximal end of the central lumen of the guide catheter then is opened, which creates a low pressure outlet for blood and establishes retrograde flow allowing any debris resulting from the dilatation to flow away from the Circle of Willis and other critical arteries and down the central lumen of the guide catheter. The dilatation balloon in the internal carotid artery then is re-inflated and both the occluding balloon in the common carotid artery and dilatation balloon in the external carotid artery are deflated to establish antegrade flow in these vessels. Any debris which remains at the bifurcation will be flushed distally through the external carotid artery by the antegrade flow. Once the debris has been flushed, the dilatation balloon in the internal carotid artery is deflated and removed, reestablishing all normal antegrade flow.

The catheter of the present invention, can further be used in a method for treating a stenosis located only in the internal carotid artery, but at a location close to the bifurcation. The occlusion guide catheter is advanced in the common carotid artery to a location proximal to the bifurcation and the occlusion balloon attached to the guide catheter is inflated until the flow of blood through this artery is totally occluded. The direction of blood flow in the internal and external carotid artery then is determined. If blood flows retrograde in the internal carotid artery toward and distally through the external carotid artery, then the stenosis may be treated with no occlusion within the external carotid artery. Preferably, a dilatation balloon catheter is introduced through the central lumen of the guide catheter and the dilatation balloon is positioned in the internal carotid artery at the stenosis. The dilatation balloon then is inflated to treat the stenosis. Next, the dilatation balloon is deflated, and retrograde flow in the internal carotid artery will flush away any debris in the internal carotid artery safely away from the Circle of Willis and other critical arteries and distally through the external carotid artery. Upon removal of the debris, the occlusion balloon is deflated to reestablish normal antegrade flow throughout the carotid arteries. This procedure has been described using a separate guide catheter and dilatation catheter. It also could be performed using a single common catheter having attached to it the dilatation balloon and the occlusion balloon.

The catheter of the present invention, can also be used in a method for treating a stenosis located only in the internal carotid artery near the bifurcation. This method is employed when it is determined that occlusion of the common carotid artery causes the blood to flow retrograde in the external carotid artery toward and distally through the internal carotid artery. In this instance, a second occlusion balloon is positioned in the external carotid artery and is inflated to stop the retrograde flow in the external carotid artery. This allows the necessary retrograde pressure in the internal carotid artery to be established. By providing a low pressure outlet for blood, retrograde flow of blood in the internal carotid artery may be established. A low pressure outlet may be provided for example through the lumen of a guide catheter carrying the balloon occluding the common carotid artery. Debris caused by treatment of the stenosis in the internal carotid artery then may be flushed safely away from the Circle of Willis and other critical arteries through the catheter.

The catheter of the present invention, can further be used in a method for treating a stenosis located solely in an internal carotid artery distal the bifurcation. In this instance, an occlusion balloon preferably carried on a guide catheter is inserted and advanced into the internal carotid artery, distal the bifurcation but proximal of the stenosis. Once the occlusion balloon is inflated, antegrade flow in the internal carotid artery ceases. An intervention component such as a dilatation catheter then is inserted via a lumen in the guide catheter into the internal carotid artery distal of the occlusion balloon and is positioned at the stenosis. After treatment of the stenosis, the dilatation catheter may be removed. Then, by providing a low pressure outlet for the internal carotid artery (such as by opening of a valve at the proximal end of the guide catheter), blood will flow retrograde in the internal carotid artery and debris may be flushed safely away from the Circle of Willis and other critical arteries through the catheter.

It is an object of the invention to reduce the risk of microembolic cerebral vessel occlusion when treating a stenosed cerebral artery.

Another object of the invention is to provide devices for occluding a cerebral artery in a manner to permit the removal of debris associated with treating a stenosis.

Another object of the invention is to provide devices for treating a cerebral artery at a location close to the bifurcation of the internal carotid artery and the external carotid artery.

Another object of the invention is to provide a catheter which permits the collection and safe removal of debris when treating a stenosis at the bifurcation of the internal and external arteries.

Another object of the invention is to provide a catheter which can be used to occlude a cerebral artery in such a manner as to permit debris to be flushed out an external carotid artery.

Yet another object of the invention is to provide a catheter which can be used to occlude internal and external carotid arteries in such a manner that debris will be flushed down a central lumen of an occlusion catheter.

Brief Description of the Drawings

Figure 1A-1C illustrate views of an occlusion balloon utilized in the present invention;

Figures 2A-2H illustrate one method of use of a catheter of the present invention;

Figures 3A-3I illustrate a second method of use of a catheter of the present invention;

Figures 4A-4E illustrate a third method of use of a catheter of the present invention;

Figures 5A-5G illustrate a fourth method of use of a catheter of the present invention; and

Figures 6A-6D illustrate a fifth method of use of a catheter of the present invention.

## Detailed Description of the Preferred Embodiments

The preferred embodiments of the angioplasty system of this invention will be described with reference to the accompanying drawings.

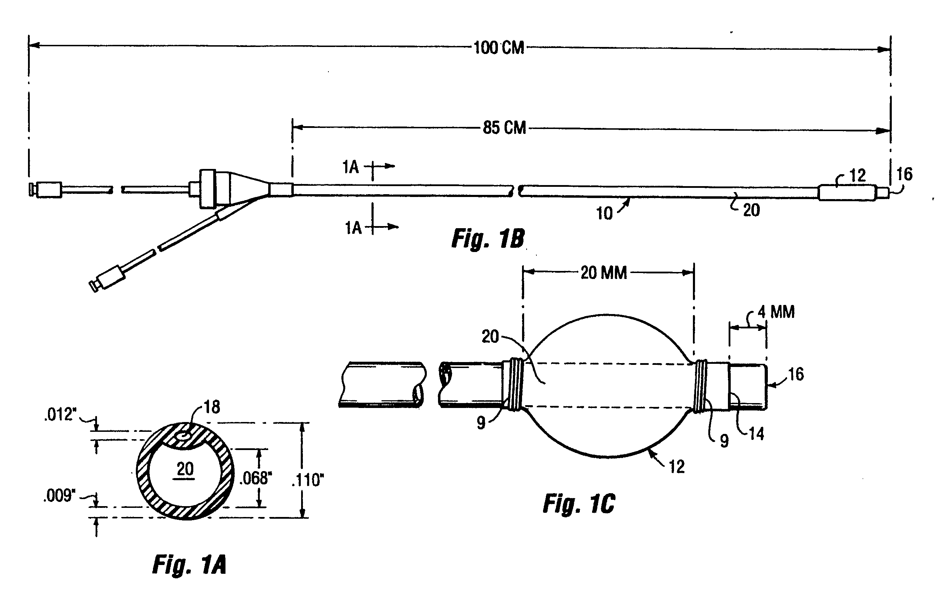

All of the described methods utilize in the preferred embodiment an occlusion catheter capable of occluding the flow of blood in a cerebral artery. Referring to Figs. 1A-1C, the preferred occlusion catheter includes a guide catheter 10 having an occlusion balloon 12 attached to its distal end. The occlusion balloon 12 is a length of tubular material coaxially disposed upon and surrounding the guide catheter 10. The occlusion balloon 12 is secured to the guide catheter 10 by dacron threads 9 wound tightly about the balloon at opposite ends. The leading edge 14 of the occlusion balloon 12 is located 4 mm from the flexible soft tip 16 of the guide catheter 10. An inflation lumen 18 extends substantially the length of the guide catheter and communicates with the interior space of the occlusion balloon 12. The guide catheter 10 also has a much larger central lumen 20 which extends the length of the guide catheter 10 and is open at either end. Preferably, the outside diameter of the guide catheter 10 is .11 inches while the inside diameter of the central lumen 20 is about .068 inches.

The minimum inner diameter of the central lumen is selected to be large enough to still permit the introduction of both a treatment device and a second occlusion device in the event that the external carotid artery must be occluded in addition to the common carotid artery. If a dilatation catheter is used to perform the second occlusion, the minimum inner diameter of the central lumen is about .038" which would be large enough to accept simultaneously a small dilatation catheter (outer diameter of .020") and a small treatment device such as a laser catheter, while still permitting blood to flow through this central lumen when the lumen is used to flush debris from a vessel. In the preferred embodiment, two dilatation catheters are passed through this lumen. In order to permit room for two small dilatation catheters and still permit the flow of blood, the inner diameter of the central lumen should be at least about 0.05". In the most preferred embodiment of the invention, the inner diameter of the central lumen is .068" which permits room for two dilatation catheters with outer diameters of .03" while still permitting flow of blood.

The preferred occlusion balloon is capable of forming a seal against a vessel wall to occlude the flow of blood in that vessel and, in particular, a cerebral vessel. Unlike a dilatation balloon, the occlusion balloon is not formed to have a fixed outside diameter upon inflation, but rather is capable of expanding at least about 500 percent, thereby allowing it to be used in vessels of varying size. Moreover, the balloon flexibly expands upon the application of relatively low pressures so that occlusion may be effected without any substantial dilatation. Preferably, sufficient expansion for occlusion is effected with less than one atmosphere of pressure, which easily may be established using a hand-held syringe. Furthermore, the balloon preferably will burst long prior to the application of pressures sufficient to cause substantial injury to a vessel wall.

The most preferred balloon is formed of latex having a thickness of not less than .004 inches, and most preferably has a thickness of about .009 inches. This balloon will expand to a maximum outer diameter of about 20 mm upon application of less than one atmosphere of pressure. Such a balloon is capable of expanding approximately 700 to 800 percent from its non-stressed resting diameter about the catheter. The balloon also may be formed from other materials which have the requisite properties, including for example silicone rubber which will expand from 600 to 700 percent of its resting diameter or polyurethane which will expand about 500 percent of its resting diameter.

The guide catheter of the invention may be used in conjunction with various treatment means or occlusion catheters depending upon the location of the stenosis, the blood flow dynamics and the dimensions of the vessel to be treated. Consequently, the guide catheter preferably is packaged separately with instructions for its use in occluding a cerebral vessel during treatment of a stenosis as described herein.

As stated in greater detail above, when treating a stenosis in a cerebral artery, care must be taken to prevent any debris or plaque from flowing distally through the internal carotid artery to narrower vessels and causing trauma. Depending upon the location of the stenosis and flow patterns resulting from the inflation of an occlusion balloon in the common carotid artery, different methods must be performed in order to prevent such trauma.

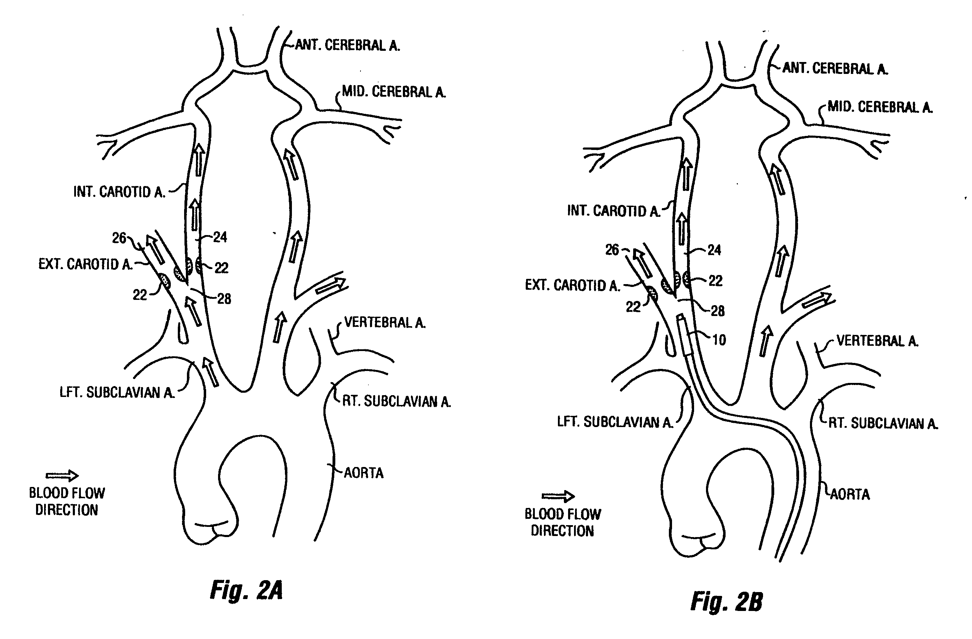

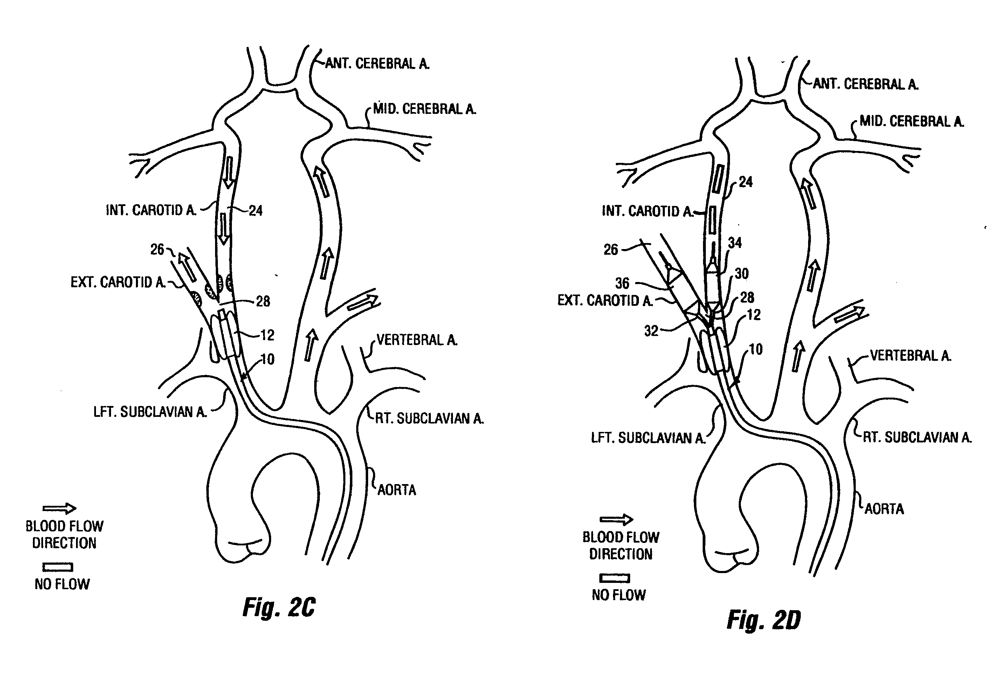

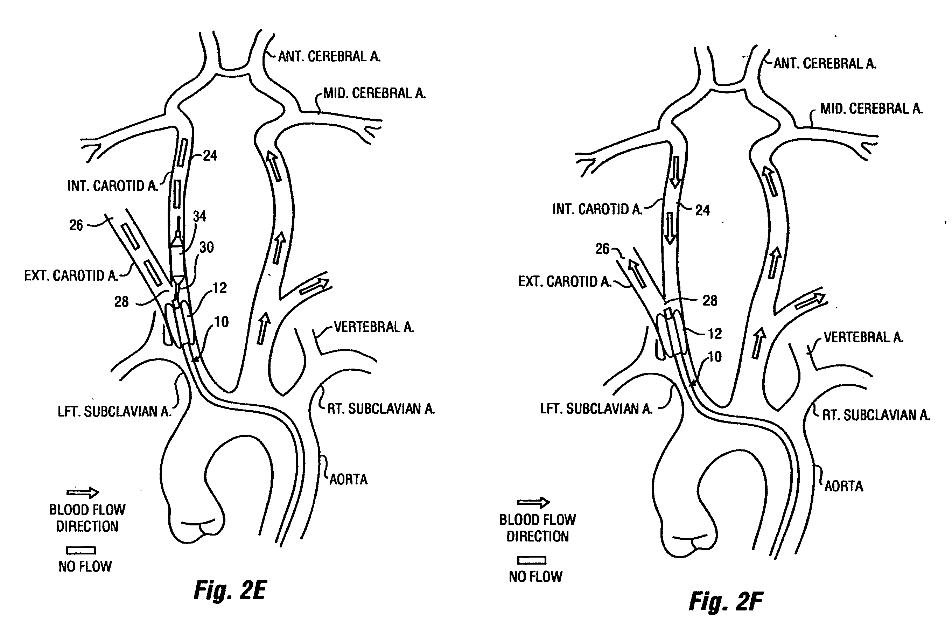

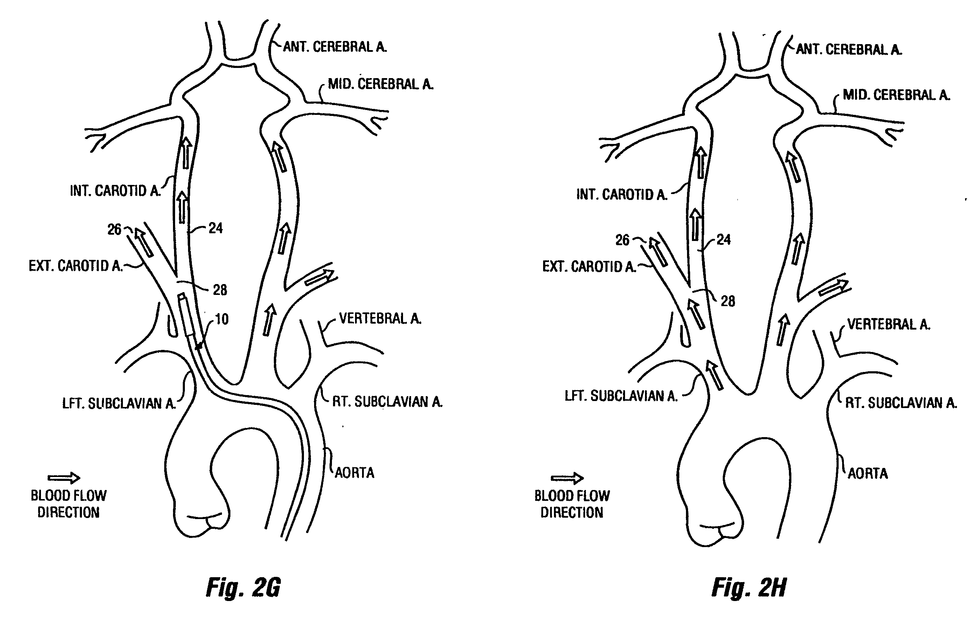

The guide catheter of the invention can be used in a variety of ways. In a first method a stenosis 22 located in both an internal carotid artery 24 and an external carotid artery 26 at or close to their bifurcation is treated. Figure 2A illustrates normal antegrade blood flow, flow direction being indicated by arrows. A guide catheter 10 with a distal occlusion balloon 12 then is advanced distally in the left subclavian artery until the occlusion balloon is positioned proximal to the bifurcation, (Figure 2B). The occlusion balloon 12 then is inflated until the artery is totally occluded (Figure 2C). It then is determined whether the blood flows retrograde in the internal carotid artery toward the external carotid artery or retrograde in the external carotid artery toward the internal artery. If it is determined that blood is flowing retrograde in the internal carotid artery toward the external carotid artery (FIG. 2C), the following procedure is followed. Dilatation balloon catheters 30 and 32 are passed through the central lumen of the guide catheter 10, one dilatation balloon 34 being positioned across the stenosis in the internal carotid artery and the other dilatation balloon 36 being positioned across the stenosis in the external carotid artery. Both dilatation balloons 34, 36 then are inflated to treat the stenoses (Figure 2D). Blood flow in all directions is stopped at this time. After the dilatation balloons have reached their desired pressures and the stenoses have been treated, the dilatation balloon 36 in the external carotid artery 26 is deflated and the dilatation catheter 32 is removed (Figure 2E). The dilatation balloon 34 in the internal carotid artery 24 then is deflated and the catheter 30 is removed, thereby reestablishing retrograde flow in the internal carotid artery 24 toward the external carotid artery 26. This flow will flush any undesirable debris safely away from the Circle of Willis and other critical arteries toward and through the external carotid artery (Figure 2F). Once the debris is flushed away, the occluding balloon 12 is deflated (Figure 2G) and removed to reestablish antegrade flow throughout the carotid arteries (Figure 2H).

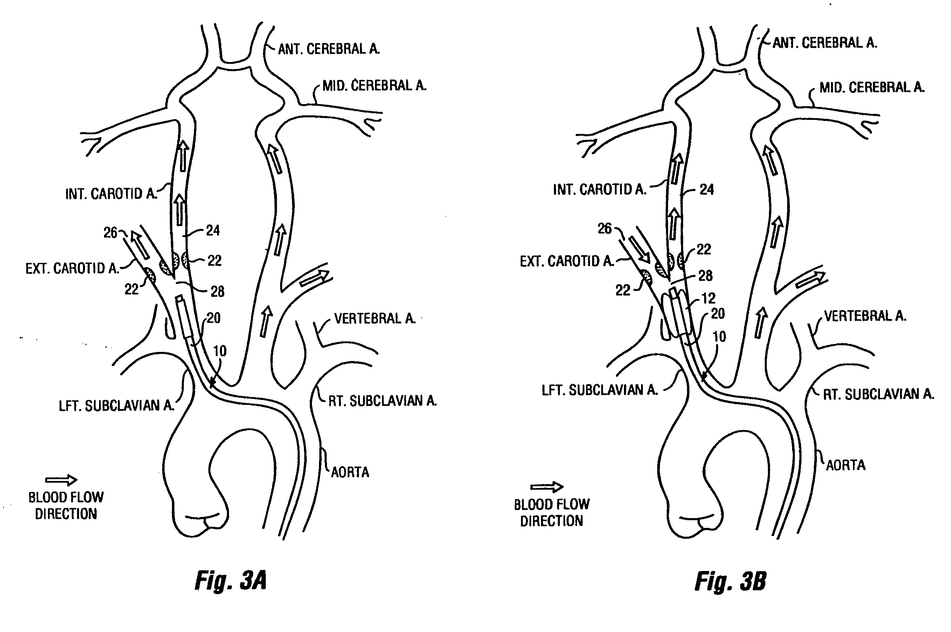

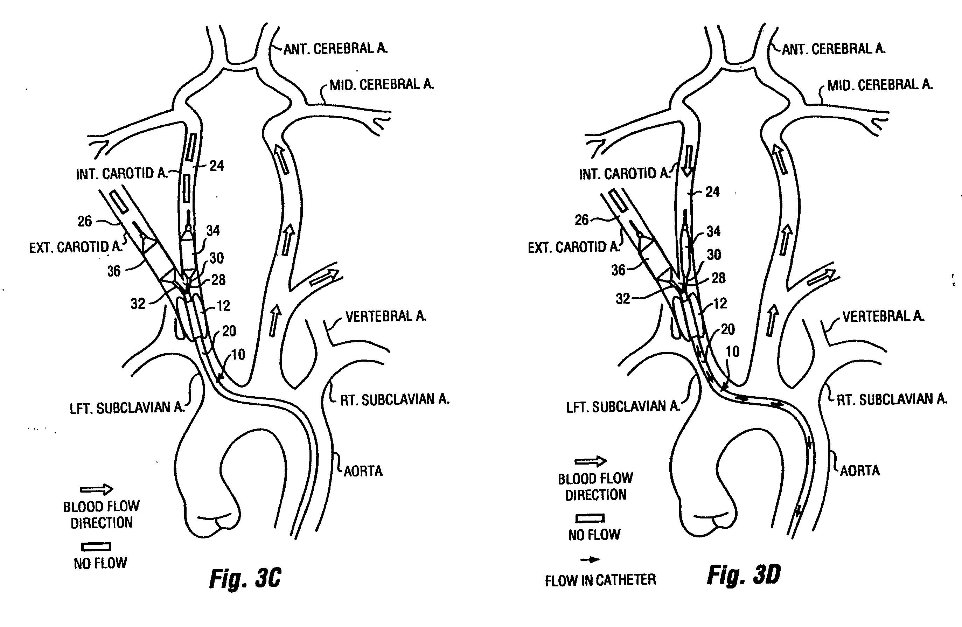

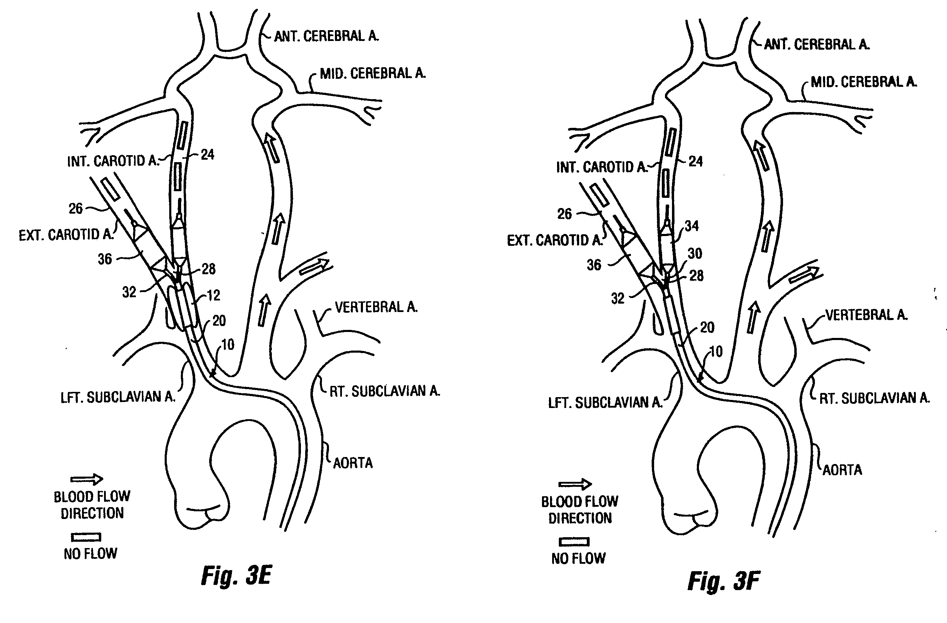

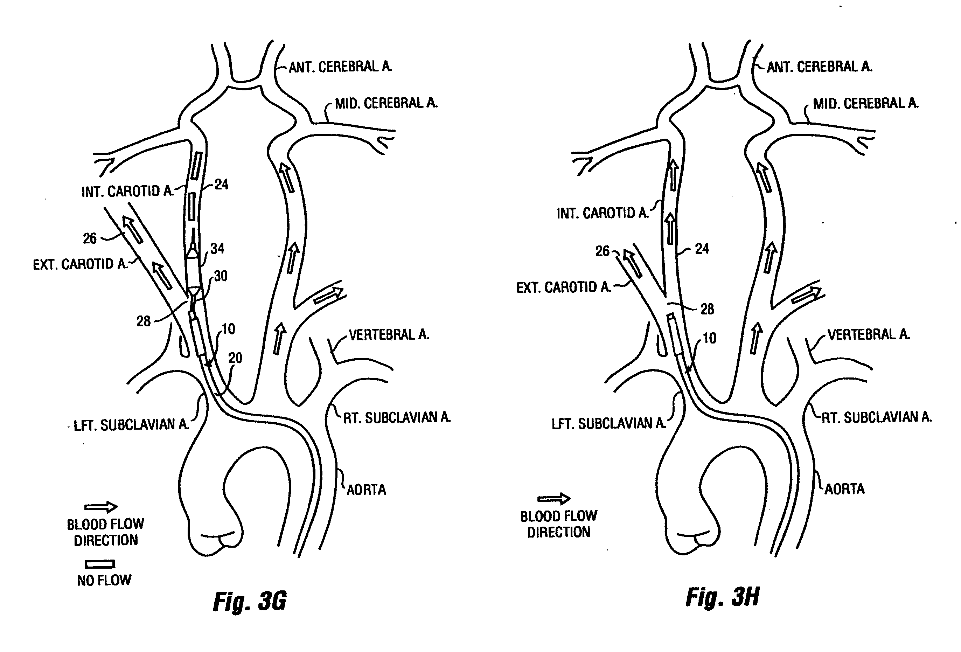

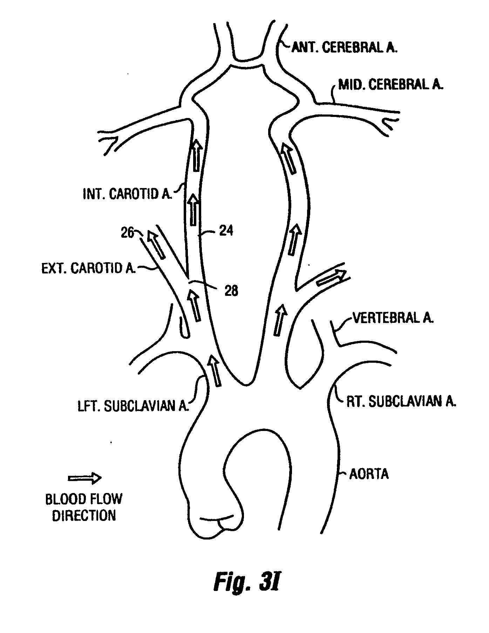

In a second method for treating a stenosis located in the external carotid artery and the internal carotid artery at or close to their bifurcation, a guide catheter 10 with a distal occlusion balloon 12 is placed in the common carotid artery proximal of the bifurcation 28 (Figure 3A) and the occlusion balloon 12 is inflated until the artery is totally occluded. If it is determined that occlusion results in blood flowing retrograde in the external carotid artery 26 and continued antegrade in the internal carotid artery 24 (Figure 3B), then the following procedure is employed. A pair of dilatation balloon catheters 30 and 32 are inserted through the central lumen of the guide catheter 10 until dilatation catheter 30 is disposed in the internal carotid artery 24 and dilatation catheter 32 is disposed in the external carotid artery 26. Both dilatation balloons 34, 36 are subsequently inflated to treat the stenoses 2 (Figure 3C). At this time, the blood is motionless at the bifurcation. After sufficient dilatation treatment, dilatation balloon 30 in the internal carotid artery 24 is deflated. Then, a valve (not shown) at the proximal end of the dilatation catheter 30 is opened to provide a low pressure outlet for the internal carotid artery 24 and to establish retrograde blood flow in the internal carotid artery 24 to flush any of the plaque that has been dislodged from the stenosis 22 down the central lumen 20 of the guide catheter 10 (Figure 3D). Once the debris has been flushed safely from the internal carotid artery 24 the dilatation balloon 30 in the internal carotid artery 24 is re-inflated (Figure 3E). Then, both the occlusion balloon 12 (Figure 3F) and the dilatation balloon 36 in the external carotid artery are deflated (Figure 3G). Any plaque remaining at the bifurcation will be flushed distally through the external carotid artery by the antegrade blood flow which is re-established. By keeping the dilatation balloon 34 in the internal carotid artery 24 inflated, the internal carotid artery 24 remains blocked to prevent any of the debris from flowing through the internal carotid artery 24. After flushing, the dilatation balloon 34 in the internal carotid artery may be deflated and removed, returning blood flow to normal antegrade flow (Figure 3H). The guide catheter 10 is withdrawn (Figure 3I).

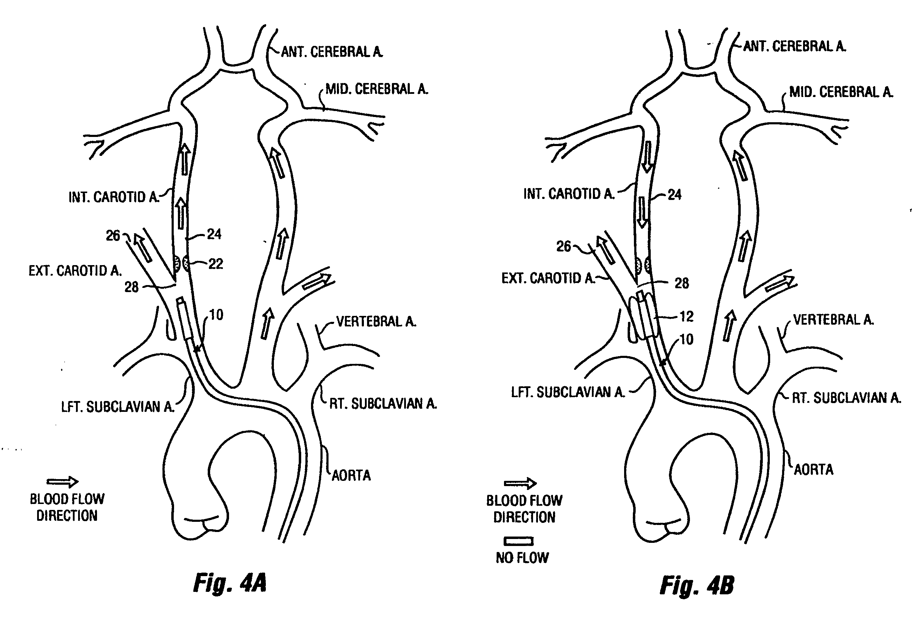

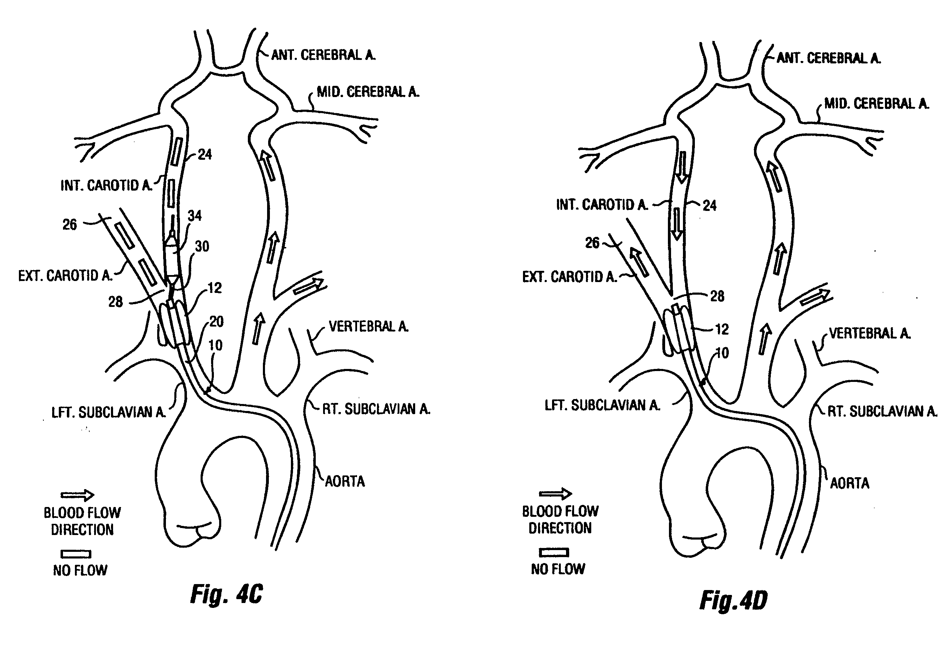

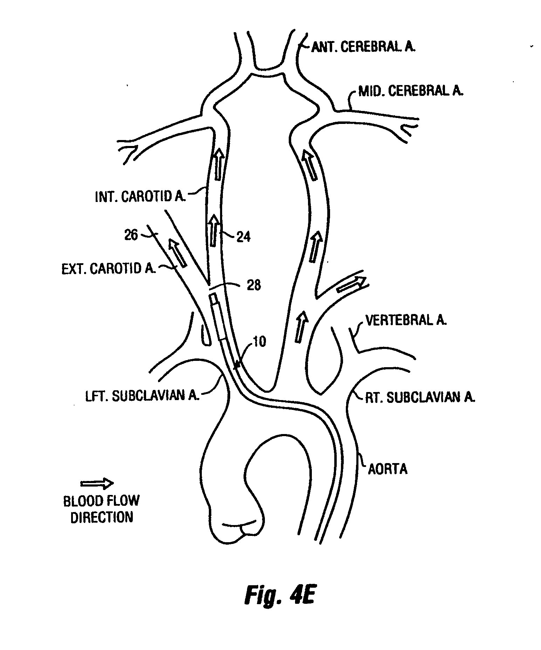

In another method for treating a stenosis located substantially in an internal carotid artery and near the bifurcation, the guide catheter 10 is placed in the common carotid artery with the occlusion balloon 12 located just proximal to the bifurcation 28 (Figure 4A). The occlusion balloon 12 is inflated until the flow of blood through the common carotid artery is totally occluded. After occluding the common carotid artery, the direction of blood flow then is determined. If it is determined that blood flows retrograde in the internal carotid artery 24 toward and distally through the external carotid artery 26 (Figure 4B) then the following procedure is employed. A dilatation catheter 30 is inserted through the central lumen 5 of the guide catheter 10 and into the internal carotid artery 24, with the dilatation balloon placed across the stenosis 22. The stenosis 22 then is treated by inflating the dilatation balloon 34. Upon the inflation of the dilatation balloon 34, the blood flow becomes motionless (bars Figure 4C). After treatment of the stenosis 22 the dilatation balloon 34 is deflated, which causes the resumption of retrograde flow in the internal carotid artery 24, thus flushing away any plaque toward and distally through the external carotid artery (Figure 4D). Once the plaque is safely removed, the occlusion balloon guide catheter 10 is removed to reestablish normal antegrade flow throughout both carotid arteries (Figure 4E).

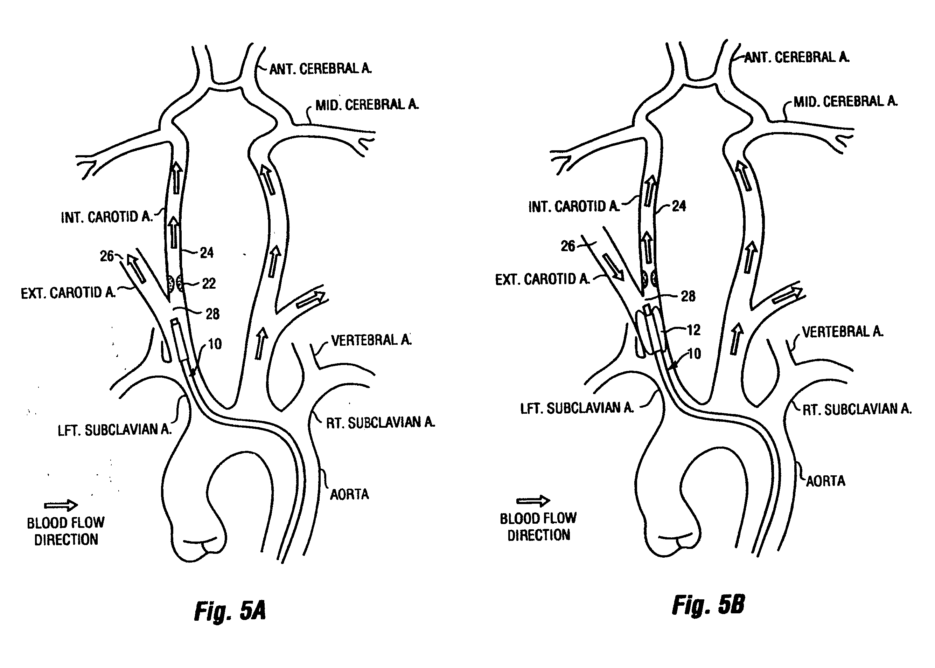

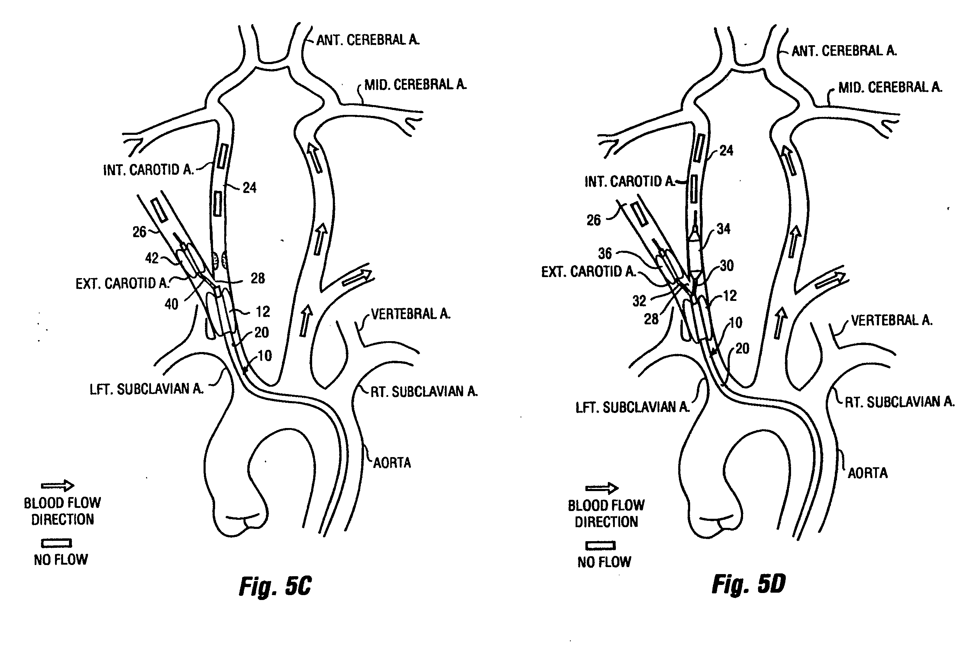

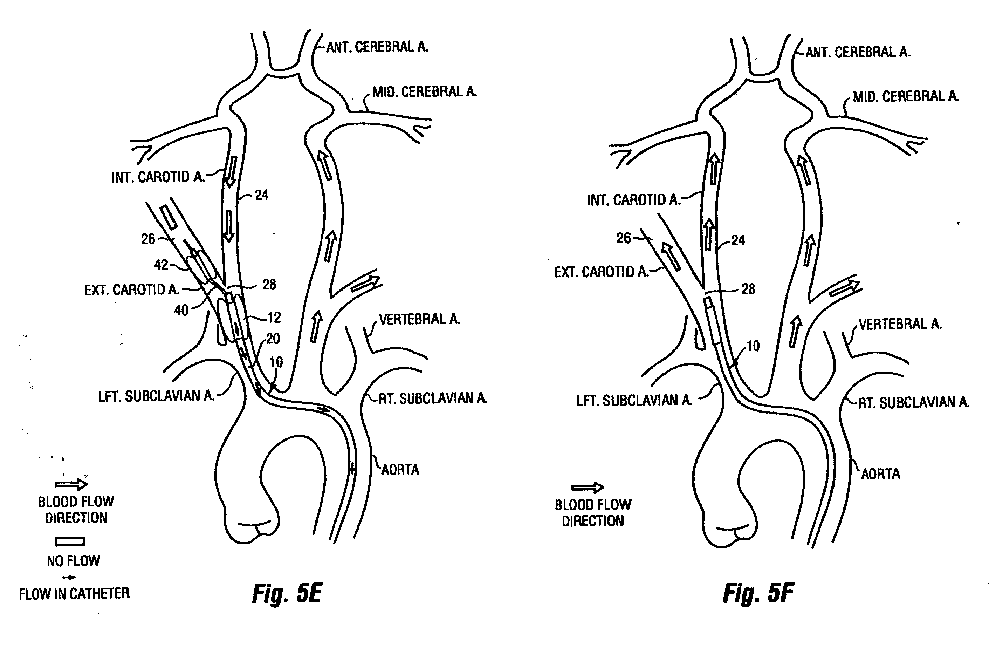

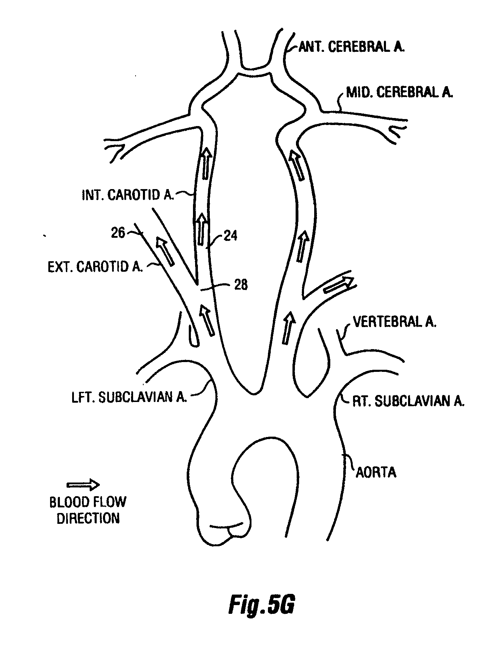

Figures 5A-5G illustrate yet another method for treating a stenosis located in the internal carotid artery close to the bifurcation, in which a guide catheter 10 is placed in the common carotid artery which the occlusion balloon 12 positioned just proximal of the bifurcation 28 Figure 5A). After inflating this occlusion balloon 12, the flow of blood through the common carotid artery is totally occluded (Figure 5B). If it is determined that blood flows retrograde in the external carotid artery 26 toward and distally through the internal carotid artery 24 (Figure 5C) then the following procedure is carried out. A catheter 40 having a second occlusion balloon 42 attached at its distal end is inserted through the central lumen 20 of the guide catheter 10 and is positioned in the external carotid artery 26. Upon inflation of the second occlusion balloon 42, blood is prevented from flowing retrograde in the external carotid artery 26, thus leaving the blood motionless (Figure 5D). Next treatment of the stenosis 22 is performed by advancing a dilatation balloon catheter 30 through the central lumen 20 of the guide catheter 10, positioning the dilatation balloon 34 in the internal carotid artery at the stenosis 22, and inflating the dilation balloon 34 (Figure 5E). After treatment, the dilatation balloon 34 is deflated and a valve (not shown) at the proximal end of the guide catheter 10 is opened to provide a low pressure outlet for the internal carotid artery 24 and to establish retrograde flow in the internal carotid artery 24. This retrograde flow flushes plaque safely out of the internal carotid artery 24 via the central lumen 20 of the guide catheter (Figure 5F). The occlusion ballon 42 of occlusion catheter 40 subsequently is deflated and removed through the central lumen 20 of the guide catheter 10. The occlusion balloon 12 of the guide catheter 10 also is deflated in order to reestablish normal antegrade flow (Figure 5G). Then, the guide catheter 10 is removed.

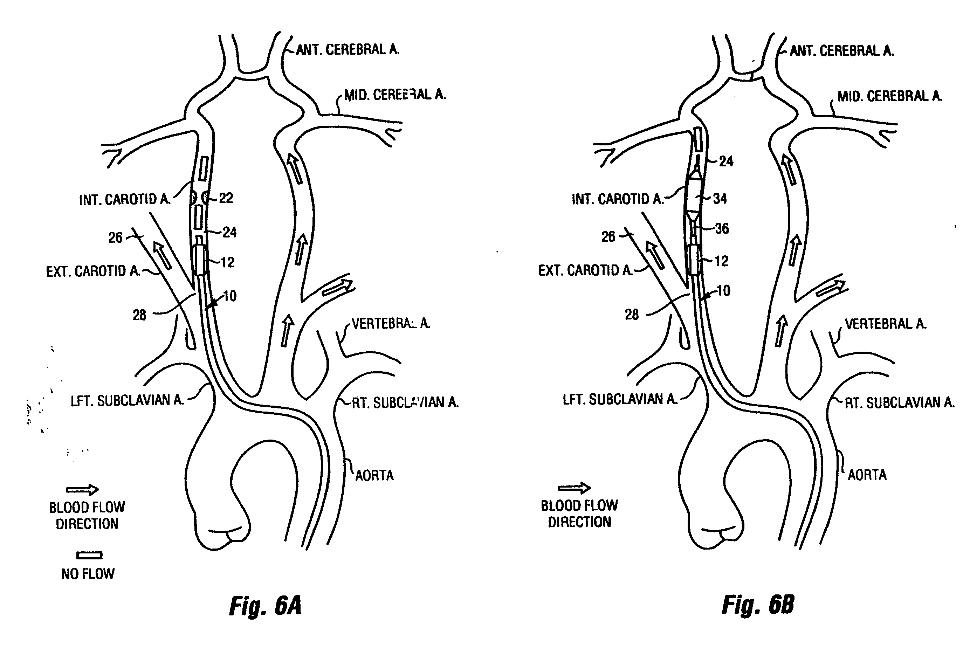

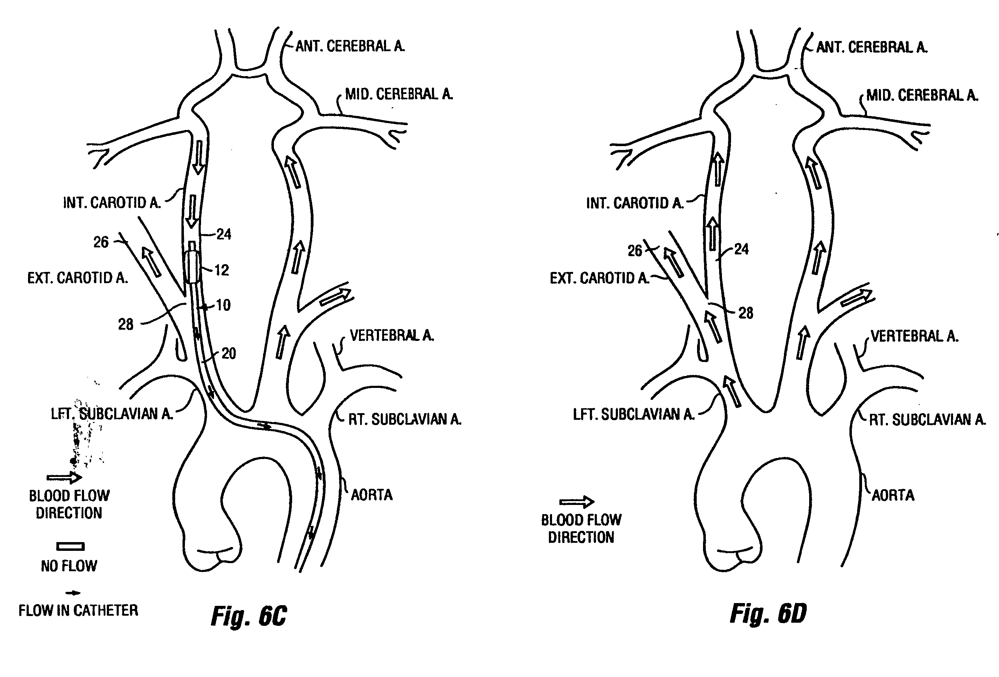

The occlusion catheter of the present invention can also be used for treating a stenosis located in the internal carotid artery distal to the bifurcation, by advancing a guide catheter 10 into the internal carotid artery until occlusion balloon 12 is positioned distal to the bifurcation. The occlusion balloon 12 then is inflated (Figure 6A.). Treatment of the stenosis 22 in the internal carotid artery 24 distal of the occlusion is performed by a dilatation catheter 30 (Figure 6B). Then, a valve (not shown) at the proximal end of the catheter is opened to provide a low pressure outlet for the blood in the internal carotid artery 24 to establish retrograde flow therein. This retrograde flow flushes debris safely from the internal carotid artery via the lumen 20 of the guide catheter (Figure 6C). Once the debris has been flushed, the occlusion balloon 12 is deflated, returning blood flow to normal antegrade flow (Figure 6D).

In the event that the stenosis occurs in the external carotid artery distal the bifurcation, the common carotid artery need not necessarily be occluded. The external carotid artery may be occluded proximal the stenosis and distal the bifurcation, while normal antegrade flow up the internal carotid artery remains uninterupted. Thus, the different methods of the present invention each prevent the flow of debris, caused by treatment of a stenosis, from flowing distally through the internal carotid artery of the patient and creating trauma.

It should be understood that the foregoing description of the invention is intended merely to be illustrative thereof and that other embodiments, modifications and equivalents may be apparent to those skilled in the art without departing from a spirit.

**Claims**

1. An occlusion catheter for use during therapeutic intervention of a blood vessel for flushing away debris, characterised by an elongated guide catheter (20) sized to be introduced into the blood vessel lumen, an occlusion balloon (12) located on said guide catheter or near the distal end of said catheter, and an inflation lumen (18) integral with said guide catheter (20) and communicating with the interior of said balloon enabling the balloon to be inflated forming a seal between the balloon and a vessel wall proximally of the therapeutic intervention, said balloon being capable of expanding to at least five times its deflated diameter when inflated.
2. An occlusion catheter as claimed in claim 1 characterised in that said lumen (18) of said guide catheter has an inner diameter of at least .068".
3. An occlusion catheter as claimed in claim 1, characterised in that said lumen (18) of said guide catheter has an inner diameter capable of permitting the passage of a dilatation balloon catheter and a treatment device.
4. A packaged catheter system for use in treating a stenosis in a cerebral vessel characterised by a guide catheter (20) having attached at its distal end an elastic occlusion balloon (12) capable of expanding to at least 500% of its deflated size without bursting, and having a guide lumen (18) extending therethrough for receiving at least one treatment device, said guide lumen having an inner diameter of at least .038"; instructions describing the use of the catheter to occlude a cerebral vessel during treatment of a stenosis, and a package containing the guide catheter and instructions.
5. A packaged catheter system as claimed in claim 4, characterised in that said inner diameter of said guide catheter (20) is at least .068".
6. An occlusion catheter for use during therapeutic intervention of a blood vessel for flushing away debris, characterised by a catheter (20), an occlusion balloon (12), attached proximally of the distal end of said catheter for occluding said blood vessel proximal a stenosis, a dilatation balloon disposed distal said occlusion balloon on said catheter for treating said stenosis, a first balloon inflation lumen for inflating said occlusion balloon, and a second inflation lumen for inflating said dilatation balloon.

100 CM

85 CM

1A →

1A →

12

16

*Fig. 1B*

10

20

20 MM

4 MM

20

16

9

14

9

12

*Fig. 1C*

.012"

18

.068"

.110"

20

.009"

*Fig. 1A*

EP 0 427 429 A2

ANT. CEREBRAL A.

MID. CEREBRAL A.

INT. CAROTID A.

26

EXT. CAROTID A.

22

24

22

28

VERTEBRAL A.

LFT. SUBCLAVIAN A.

RT. SUBCLAVIAN A.

BLOOD FLOW
DIRECTION

AORTA

*Fig. 2A*

ANT. CEREBRAL A.

MID. CEREBRAL A.

INT. CAROTID A.

26

EXT. CAROTID A.

22

24

22

28

10

VERTEBRAL A.

LFT. SUBCLAVIAN A.

RT. SUBCLAVIAN A.

BLOOD FLOW
DIRECTION

AORTA

*Fig. 2B*

EP 0 427 429 A2

ANT. CEREBRAL A.

MID. CEREBRAL A.

INT. CAROTID A.

26

EXT. CAROTID A.

VERTEBRAL A.

LFT. SUBCLAVIAN A.

RT. SUBCLAVIAN A.

AORTA

BLOOD FLOW
DIRECTION

NO FLOW

Fig. 2C

ANT. CEREBRAL A.

MID. CEREBRAL A.

INT. CAROTID A.

26

36

EXT. CAROTID A.

32

VERTEBRAL A.

LFT. SUBCLAVIAN A.

RT. SUBCLAVIAN A.

AORTA

BLOOD FLOW
DIRECTION

NO FLOW

Fig. 2D

EP 0 427 429 A2

EP 0 427 429 A2

ANT. CEREBRAL A.

MID. CEREBRAL A.

24

INT. CAROTID A.

26

34

30

EXT. CAROTID A.

28

12

10

VERTEBRAL A.

LFT. SUBCLAVIAN A.

RT. SUBCLAVIAN A.

AORTA

⟹ BLOOD FLOW DIRECTION

▭ NO FLOW

**Fig. 2E**

ANT. CEREBRAL A.

MID. CEREBRAL A.

INT. CAROTID A.

24

26

EXT. CAROTID A.

28

12

10

VERTEBRAL A.

LFT. SUBCLAVIAN A.

RT. SUBCLAVIAN A.

AORTA

⟹ BLOOD FLOW DIRECTION

▭ NO FLOW

**Fig. 2F**

**Fig. 2G**

ANT. CEREBRAL A.

MID. CEREBRAL A.

INT. CAROTID A.

26

24

EXT. CAROTID A.

28

10

VERTEBRAL A.

LFT. SUBCLAVIAN A.

RT. SUBCLAVIAN A.

AORTA

BLOOD FLOW
DIRECTION

**Fig. 2H**

ANT. CEREBRAL A.

MID. CEREBRAL A.

INT. CAROTID A.

26

24

EXT. CAROTID A.

28

VERTEBRAL A.

LFT. SUBCLAVIAN A.

RT. SUBCLAVIAN A.

AORTA

BLOOD FLOW
DIRECTION

EP 0 427 429 A2

Fig. 3A

Fig. 3B

**Fig. 3C**

ANT. CEREBRAL A.

MID. CEREBRAL A.

INT. CAROTID A.

24

26

EXT. CAROTID A.

34

36

30

28

32

12

20

10

VERTEBRAL A.

LFT. SUBCLAVIAN A.

RT. SUBCLAVIAN A.

AORTA

⇨ BLOOD FLOW DIRECTION

▭ NO FLOW

**Fig. 3D**

ANT. CEREBRAL A.

MID. CEREBRAL A.

INT. CAROTID A.

24

26

EXT. CAROTID A.

34

36

30

28

32

12

20

10

VERTEBRAL A.

LFT. SUBCLAVIAN A.

RT. SUBCLAVIAN A.

AORTA

⇨ BLOOD FLOW DIRECTION

▭ NO FLOW

→ FLOW IN CATHETER

EP 0 427 429 A2

**Fig. 3E**

**Fig. 3F**

EP 0 427 429 A2

INT. CAROTID A.

26

EXT. CAROTID A.

28

24

34

30

10

20

ANT. CEREBRAL A.

MID. CEREBRAL A.

VERTEBRAL A.

LFT. SUBCLAVIAN A.

RT. SUBCLAVIAN A.

AORTA

⇒ BLOOD FLOW
DIRECTION

▭ NO FLOW

*Fig. 3G*

INT. CAROTID A.

26

EXT. CAROTID A.

24

28

10

ANT. CEREBRAL A.

MID. CEREBRAL A.

VERTEBRAL A.

LFT. SUBCLAVIAN A.

RT. SUBCLAVIAN A.

AORTA

⇒ BLOOD FLOW
DIRECTION

*Fig. 3H*

EP 0 427 429 A2

*Fig. 3I*

ANT. CEREBRAL A.

MID. CEREBRAL A.

INT. CAROTID A.

26

24

EXT. CAROTID A.

28

VERTEBRAL A.

LFT. SUBCLAVIAN A.

RT. SUBCLAVIAN A.

AORTA

BLOOD FLOW
DIRECTION

EP 0 427 429 A2

**Fig. 4A**

ANT. CEREBRAL A.

MID. CEREBRAL A.

INT. CAROTID A.

26
24
22
EXT. CAROTID A.
28
10

VERTEBRAL A.

LFT. SUBCLAVIAN A.

RT. SUBCLAVIAN A.

AORTA

⟹ BLOOD FLOW DIRECTION

**Fig. 4B**

ANT. CEREBRAL A.

MID. CEREBRAL A.

INT. CAROTID A. — 24

26
EXT. CAROTID A.
28
12
10

VERTEBRAL A.

LFT. SUBCLAVIAN A.

RT. SUBCLAVIAN A.

AORTA

⟹ BLOOD FLOW DIRECTION

▭ NO FLOW

EP 0 427 429 A2

ANT. CEREBRAL A.

MID. CEREBRAL A.

INT. CAROTID A.

24

26

34

30

EXT. CAROTID A.

28

12

20

10

VERTEBRAL A.

LFT. SUBCLAVIAN A.

RT. SUBCLAVIAN A.

AORTA

⇒ BLOOD FLOW DIRECTION

▭ NO FLOW

*Fig. 4C*

ANT. CEREBRAL A.

MID. CEREBRAL A.

INT. CAROTID A.

24

26

EXT. CAROTID A.

28

12

10

VERTEBRAL A.

LFT. SUBCLAVIAN A.

RT. SUBCLAVIAN A.

AORTA

⇒ BLOOD FLOW DIRECTION

▭ NO FLOW

*Fig.4D*

EP 0 427 429 A2

ANT. CEREBRAL A.

MID. CEREBRAL A.

INT. CAROTID A.

26

24

EXT. CAROTID A.

28

10

VERTEBRAL A.

LFT. SUBCLAVIAN A.

RT. SUBCLAVIAN A.

AORTA

BLOOD FLOW
DIRECTION

*Fig. 4E*

EP 0 427 429 A2

ANT. CEREBRAL A.

MID. CEREBRAL A.

VERTEBRAL A.

RT. SUBCLAVIAN A.

AORTA

INT. CAROTID A.

EXT. CAROTID A.

LFT. SUBCLAVIAN A.

24

28

12

10

26

BLOOD FLOW
DIRECTION

*Fig. 5B*

ANT. CEREBRAL A.

MID. CEREBRAL A.

VERTEBRAL A.

RT. SUBCLAVIAN A.

AORTA

INT. CAROTID A.

EXT. CAROTID A.

LFT. SUBCLAVIAN A.

24

22

28

10

26

BLOOD FLOW
DIRECTION

*Fig. 5A*

22

Fig. 5D

Fig. 5C

Fig. 5F

Fig. 5E

24

ANT. CEREBRAL A.

MID. CEREBRAL A.

VERTEBRAL A.

RT. SUBCLAVIAN A.

AORTA

INT. CAROTID A.

EXT. CAROTID A.

LFT. SUBCLAVIAN A.

24

26

28

BLOOD FLOW
DIRECTION

*Fig.5G*

**Fig. 6A**

ANT. CEREBRAL A.

MID. CEREBRAL A.

INT. CAROTID A.

26

EXT. CAROTID A.

28

LFT. SUBCLAVIAN A.

VERTEBRAL A.

RT. SUBCLAVIAN A.

AORTA

BLOOD FLOW
DIRECTION

NO FLOW

22
24
12
10

**Fig. 6B**

ANT. CEREBRAL A.

MID. CEREBRAL A.

INT. CAROTID A.

26

EXT. CAROTID A.

28

LFT. SUBCLAVIAN A.

VERTEBRAL A.

RT. SUBCLAVIAN A.

AORTA

BLOOD FLOW
DIRECTION

24
34
36
12
10

EP 0 427 429 A2

*Fig. 6D*

*Fig. 6C*

ANT. CEREBRAL A.
MID. CEREBRAL A.
VERTEBRAL A.
RT. SUBCLAVIAN A.
AORTA
INT. CAROTID A.
EXT. CAROTID A.
LFT. SUBCLAVIAN A.
BLOOD FLOW DIRECTION
NO FLOW
FLOW IN CATHETER